# EUROPÄISCHE PATENTSCHRIFT

(11) **EP 0 941 980 B1**
(45) Veröffentlichungstag und Bekanntmachung des Hinweises auf die Patenterteilung: **28.11.2001**
(21) Anmeldenummer: 99104436.3
(22) Anmeldetag: 05.03.1999
(51) Int. Cl.: C07C 45/74, C07C 49/603

(54) **Verfahren zur Herstellung von 2,5,6-Trimethyl-cyclohex-2-en-1-on**
Process for the preparation of 2,5,6-trimethyl-cyclohex-2-en-1-on
Procédé pour la préparation de 2,5,6-triméthyl-cyclohex-2-ène-1-one

(30) Priorität: 12.03.1998 DE 19810671
(43) Veröffentlichungstag der Anmeldung: 15.09.1999
(73) Patentinhaber: BASF AKTIENGESELLSCHAFT, 67056 Ludwigshafen (DE)
(72) Erfinder: Ruff, Detlef, Dr., 67067 Ludwigshafen (DE); Burst, Wolfram, 68199 Mannheim (DE); Kaiser, Wulf, Dr., 67098 Bad Dürkheim (DE); Stroezel, Manfred, 68549 Ilvesheim (DE)

(56) Entgegenhaltungen:
- DE-B- 1 793 037
- GB-A- 852 935
- US-A- 3 857 892

## Beschreibung

Die Erfindung betrifft ein verbessertes Verfahren zur Herstellung von 2,5,6-Trimethyl-cyclohex-2-en-1-on. Das Verfahrensprodukt ist von großer technischer Bedeutung, da es technisch leicht zu 2,3,6-Trimethyl-phenol dehydriert werden kann, welches ein wichtiges Vorprodukt für die Synthese von Vitamin E ist.

Aus DE 1 793 037 ist ein Verfahren zur Herstellung von 2,5,6-Trimethyl-cyclohex-2-en-1-on durch Umsetzung von Diethylketon mit Crotonaldehyd oder einer Verbindung, die unter den gewählten Reaktionsbedingungen in Crotonaldehyd umgewandelt wird, in Gegenwart von basischen Verbindungen, anschließende Neutralisation des Reaktionsgemisches und dessen destillative Aufarbeitung bekannt. Das Auffinden dieser einfachen Herstellungsmöglichkeit für Trimethylcyclohexenon und damit für Trimethylphenol war seinerzeit eine enorme Verbesserung der synthetischen Herstellung von Vitamin E, da bis dahin Trimethylphenol nur durch die schwierige und unselektive Alkylierung von Phenol herstellbar war. Die Ausbeuten dieses an sich sehr guten Verfahrens lassen jedoch noch zu wünschen übrig; sie liegen nachweislich der Beispiele zwischen 40% und 77%. Ein weiterer Nachteil dieses Verfahrens ist, daß es bislang nur diskontinuierlich durchgeführt werden konnte, was bei einer großtechnischen Herstellung zu sehr großen Reaktionsgefäßen und damit zu sehr kleinen Raum-Zeit-Ausbeuten führt. Da diese Umsetzung mit α,β-ungesättigten Aldehyden immer in großer Verdünnung durchgeführt werden muß, arbeitet man also ständig mit sehr großen Mengen siedender Lösungsmittel, was aus sicherheitstechnischen Gründen unerwünscht ist.

In DE 3 636 057 ist ein Verfahren zur Herstellung von 2,5,6-Trimethyl-cyclohex-2-en-1-on beschrieben, bei dem man die gemäß DE 1 793 037 erzielbaren Ausbeuten dadurch verbessern kann, daß man die bei der destillativen Aufarbeitung anfallenden Destillationsrückstände ggf in Gegenwart katalytischer Mengen Alkali auf Temperaturen von 120 bis 250°C erhitzt.

Weiterhin ist aus JP 58-035141 A2 ein Verfahren zur Herstellung von Cyclohex-2-en-1-onen durch Umsetzen von α,β-ungesättigten Aldehyden mit aliphatischen Ketonen in der Gasphase beschrieben. Als Vorteil gegenüber der Umsetzung in der flüssigen Phase wird eine Unterdrückung der Polymerisation der α,β-ungesättigten Aldehyde, wie Acrolein oder Crotonaldehyd, genannt. Die erzielten Ausbeuten für 2,5,6-Trimethyl-cyclohex-2-en-1-on liegen jedoch in den entsprechenden Beispielen für die Umsetzung von Crotonaldehyd auch nur zwischen 57 und 76% der Theorie.

Die in DE 1 793 037 beschriebene Umsetzung wurde bislang auch in industriellem Maßstab als diskontinuierliches Verfahren betrieben, in welchem zu einer vorgelegten Mischung aus Diethylketon, Base und ggf. einem Lösungsmittel Crotonaldehyd zugetropft wurde, wobei es als vorteilhaft angesehen wurde wenn der Crotonaldehyd oder dessen Vorstufe vor dem Zutropfen durch ein Lösungsmittel verdünnt wurde. Zur Vervollständigung der Umsetzung wurde nach beendeter Crotonaldehyd-Zugabe das Reaktionsgemisch für eine gewisse Zeit weiter erhitzt.

Eine kontinuierliche Verfahrensführung wurde nicht für möglich gehalten, da zu erwarten war, daß bei einer kontinuierlichen Verfahrensführung der Reaktion ein Großteil des Crotonaldehyds aufgrund seiner erhöhten Konzentration in der Reaktionsmischung durch Selbstpolymerisation Nebenreaktionen eingeht. Auch wäre zu erwarten gewesen, daß zur Gewährleistung einer Nachreaktionszeit eine ganze Kaskade von Reaktoren erforderlich sei.

Die bislang beste Reaktionsführung sieht Reaktionszeiten von einer Stunde vor. Die Kondensation wird im allgemeinen bei Temperaturen zwischen -20°C und 200°C, vorzugsweise zwischen 20 und 120°C ausgeführt (vgl. DE 1 793 037, Beispiel 1). An sich war zu erwarten, daß die Reaktionsmischung durch Wärmebehandlung über den beschriebenen Bereich hinaus auf Grund von Folgereaktionen verharzt.

In DE 3 636 057 wird beschrieben, daß die maximale Grenztemperatur der Behandlung des Reaktionsaustrages bei 220°C liegt und daß nur bei Zusatz von inerten Hochsiedern eine Behandlung bis 250°C möglich ist.

Es war also zu erwarten, daß bei höheren Temperaturen als den bislang beschriebenen eine vollständige Polymerisation des α,β-ungesättigten Aldehyds oder des gesamten Reaktionsaustrages eintritt.

Es war daher die Aufgabe der Erfindung, Bedingungen zu finden, unter denen man die Herstellung von 2,5,6-Trimethyl-cyclohex-2-en-1-on, durch Umsetzen von Diethylketon mit Crotonaldehyd in industriellem Maßstab mit besseren Ausbeuten und besseren Raum-Zeit-Ausbeuten durchführen kann. Dies bedeutete praktisch, Bedingungen zu finden, unter denen die genannte Umsetzung kontinuierlich durchgeführt werden kann, und dies möglichst unter Verbesserung der chemischen Ausbeute und der Qualität des 2,5,6-Trimethyl-cyclohex-2-en-1-ons.

Überraschenderweise wurde gefunden, daß die Umsetzung von Diethylketon mit Crotonaldehyd in Gegenwart von Basen sehr vorteilhaft auch kontinuierlich durchgeführt werden kann, wenn man gleichzeitig überschüssiges Diethylketon, den Crotonaldehyd und die Lösung oder Suspension einer starken Base kontinuierlich in einen gegebenenfalls erhitzten Druckkessel mit intensiver Vermischung so zupumpt, daß die Reaktionstemperatur zwischen 150 und 350°C liegt und die mittlere Verweilzeit im Druckkessel nur 0,1 Sekunde bis 60 Sekunden beträgt.

Überraschenderweise wurde auch festgestellt, daß man bei Einhaltung besonders geeigneter Verweilzeiten bei der kontinuierlichen Durchführung der Umsetzung auf eine Vorverdünnung des Crotonaldehyds mit inerten Hochsiedern verzichten kann und daß man die kontinuierliche Synthese in einem einzigen Reaktionskessel durchführen kann. Dies bedeutet, daß die Verwendung von mehreren hintereinandergeschalteten Reaktionsgefäßen nicht notwendig ist. Darüberhinaus hat sich gezeigt, daß bei kontinuierlicher Verfahrensführung sogar die Menge an hochsiedenden Nebenprodukten durch geeignete Wahl der Verweilzeit erheblich gesenkt werden kann.

Gegenstand der Erfindung ist daher ein Verfahren zur Herstellung von 2,5,6-Trimethyl-cyclohex-2-en-1-on durch Umsetzen von Diethylketon in Gegenwart von basischen Mitteln mit Crotonaldehyd oder einer Verbindung, die unter den gewählten Reaktionsbedingungen in Crotonaldehyd umgewandelt wird, bei erhöhter Temperatur, das dadurch gekennzeichnet ist, daß man
a) gleichzeitig ein Mol Crotonaldehyd oder 1 Mol einer Verbindung, die unter den Reaktionsbedingungen in Crotonaldehyd umgewandelt wird, 5 bis 30 Mole Diethylketon und die Lösung oder Suspension einer starken Base durch getrennte Zuleitungen oder zumindest teilweise in Form eines Gemisches kontinuierlich in einen gegebenenfalls erhitzten Druckbehälter, der eine intensive Vermischung gewährleistet, so zupumpt, daß
b) die Reaktionstemperatur zwischen 150°C und 350°C liegt und die mittlere Verweilzeit im Druckbehälter 0,1 Sekunde bis 20 Minuten, vorzugsweise 0,1 Sekunde bis 15 Minuten, insbesondere 0,1 Sekunden bis 60 Sekunden, beträgt.

Mit Vorteil geht man bei dem erfindungsgemäßen Verfahren so vor, daß man gleichzeitig den Crotonaldehyd,das Diethylketon und die Lösung der starken Base so in den Druckbehälter pumpt, daß die Reaktionstemperatur 200 bis 300°C, vorzugsweise 220 bis 290°C, insbesondere 225 bis 280°C beträgt. Hierbei ist es möglich, den Druckbehälter selbst zu erhitzen und die Reaktionskomponenten ohne vorheriges Erwärmen einzutragen. Man kann aber auch die für die Umsetzung notwendige Energie zumindest teilweise durch Vorerhitzen der Reaktionskomponenten in das Reaktionsgefäß einbringen.

Zur Durchführung des erfindungsgemäßen Verfahrens ist es möglich, die 3 Reaktionskomponenten, den Crotonaldehyd, bzw. die Verbindung, die unter den Reaktionsbedingungen in Crotonaldehyd umgewandelt wird, das Diethylketon und die Lösung der starken Base gleichzeitig, aber durch getrennte Zuleitungen in den Druckbehälter einzutragen. Man kann aber auch die Reaktionskomponenten zumindest teilweise in Form eines Gemisches in den Druckbehälter eintragen, wenn man darauf achtet, daß nicht der bekanntermaßen in alkalischem Milieu zu Polymerisationen neigende Crotonaldehyd vor der Umsetzung mit der Lösung oder Suspensionen der starken Base zusammenkommt. So kann man beispielsweise ein Gemisch aus Crotonaldehyd und Diethylketon durch eine Zuleitung einführen und die Lösung der Base durch eine weitere Zuleitung. Man kann aber auch das Diethylketon und die Lösung der Base zu einer Emulsion vermischen und diese durch eine Zuleitung eintragen und den Crotonaldehyd durch eine zweite. Je nach Gestaltung der Reaktionsanlagen kann es zweckmäßig sein, daß man den Crotonaldehyd mit einem Teil des Diethylketons vermischt einträgt, die Lösung der Base durch eine zweite Zuleitung und den Rest des Diethylketons vorerhitzt durch eine dritte Zuleitung in das Druckgefäß einträgt.

Das Diethylketon verwendet man im allgemeinen in Mengen von 5 bis 30 Mol, vorzugsweise 10 bis 20 Mol, pro Mol Crotonaldehyd oder pro Mol einer Verbindung, die unter den Reaktionsbedingungen in Crotonaldehyd umgewandelt wird. Man kann zwar Diethylketon auch in noch größeren Mengen einsetzen, dies bringt jedoch keine Vorteile, sondern erschwert nur unnötig die Aufarbeitung des Reaktionsansatzes und erhöht unnötig die Energiekosten für die destillative Aufarbeitung.

Als stark basische Mittel können in dem erfindungsgemäßen Verfahren Hydroxide, Oxide, Alkoholate, Amide, Hydride, Carbonate oder metallorganische Verbindungen der Alkali- oder Erdalkalimetalle oder quartäre Ammoniumhydroxide verwendet werden. Es seien beispielsweise genannt : Natrium-, Kalium- oder Calciumhydroxid, Natriumcarbonat, Kaliumcarbonat, Natriummethylat, Natriumamid, Natriumhydrid, Phenyllithium, Methyllithium und Tetramethylammoniumhydroxid. Im allgemeinen ist es vorteilhaft, wenn man die starke Base in Form einer Lösung oder einer Suspension in das Reaktionsgefäß einbringt.

Mit Vorteil gelingt das erfindungsgemäße Verfahren, wenn man als starke Basen Alkalialkoholate in den entsprechenden Alkanolen oder aber Alkalihydroxide in wässriger Lösung verwendet. Die Verwendung wäßriger Lösungen bringt den großen Vorteil mit sich, daß durch die getrennten Phasen mit Beendigung der intensiven Durchmischung der Prozeß abgebrochen wird, ohne daß eine Neutralisation notwendig wird.

Mit besonderem Vorteil verwendet man daher eine konzentrierte wässrige Lösung eines Alkalihydroxids, insbesondere von KOH, d.h. eine wäßrige Lösung des Alkalihydroxids, insbesondere des Kaliumhydroxids, in der das Alkalihydroxid, insbesondere das KOH in einer Konzentration von 30 bis 70 Gew.-%, vorliegt. Bei Verwendung von noch höher konzentrierten wäßrigen Lösungen wären beheizte Leitungen und Pumpen notwendig. Zweckmäßig verwendet man die konzentrierte, etwa 48 bis 50 Gew.-%ige wäßrige Lösung von KOH, wie sie kommerziell erhältlich ist.

Die starke Base verwendet man im allgemeinen in Mengen von 0,02 bis 3 Mol pro Mol Crotonaldehyd. Die Menge der starken Base hängt weitgehend von der Intensität der Vermischung im Druckbehälter ab.

Die erfindungsgemäße Umsetzung wird im allgemeinen in einem Druckbereich durchgeführt, der etwas über dem Eigendruck des Reaktionsgemisches liegt, d.h. in einem Druckbereich, in dem die Reaktionskomponenten bei gegebener Temperatur flüssig vorliegen und ein Sieden verhindert wird.

Die Aufarbeitung der Reaktionsmischung kann wie in DE 1 793 037 beschrieben durchgeführt werden.

Mit Hilfe des erfindungsgemäßen Verfahrens ist es möglich, das als Zwischenprodukt für die Herstellung von Vitamin E essentielle 2,5,6-Trimethyl-cyclohex-2-en-1-on auch in industriellem Maßstab auf technisch einfache Weise in sehr guten Ausbeuten und sehr guten Raum-Zeit-Ausbeuten herzustellen.

### Beispiele 1-3

Eine Mischung aus jeweils einem Mol Crotonaldehyd und 16 Molen Diethylketon wurde kontinuierlich in einen Druckbehälter unter intensiver Durchmischung so zugepumpt, daß die Reaktionstemperatur, bzw. die mittlere Verweilzeit den aus der Tabelle 1 ersichtlichen Werten entsprach. Gleichzeitig wurde über eine weitere Zuführung jeweils die aus der Tabelle 1 ersichtliche Anzahl von Molen KOH pro Mol Crotonaldehyd in Form einer konzentrierten wässrigen Lösung in diesen Druckbehälter mit intensiver Durchmischung zudosiert. Über ein Austragsventil wurde ein über dem Eigendruck der Reaktionsmischung liegender Systemdruck eingestellt. Der Reaktionsaustrag wurde auf Raumtemperatur (RT) abgekühlt. Die Ausbeute wurde durch Gaschromatographie mit internem Standard (GC) bestimmt. In der folgenden Tabelle wird die jeweils erzielte Ausbeute an 2,5,6-Trimethyl-cyclohex-2-en-1-on, bezogen auf eingesetzten Crotonaldehyd, angegeben.

**Tabelle**

| Beispiel | Reaktionstemperatur [°C] | Verweilzeit | Menge KOH [Mol] | Ausbeute [% der Theorie] |
|---|---|---|---|---|
| 1 | 237 | 10 min | 0,3 | 78 |
| 2 | 277 | 10 min | 0,3 | 82 |
| 3 | 269 | 2 min | 0,3 | 83 |
| 4 | 242 | 5 sec | 0,2 | 86 |
| 5 | 268 | 5 sec | 0,2 | 92 |

## Patentansprüche

1. Verfahren zur Herstellung von 2,5,6-Trimethyl-cyclohex-2-en-1-on durch Umsetzen von Diethylketon mit Crotonaldehyd oder einer Verbindung, die unter den gewählten Reaktionsbedingungen in Crotonaldehyd umgewandelt wird, in Gegenwart von basischen Mitteln bei erhöhter Temperatur, **dadurch gekennzeichnet, daß** man
a) gleichzeitig ein Mol Crotonaldehyd oder ein Mol einer Verbindung, die unter den Reaktionsbedingungen in Crotonaldehyd umgewandelt wird, 5 bis 30 Mole Diethylketon und die wässrige Lösung oder Suspension einer starken Base durch getrennte Zuleitungen oder zumindest teilweise in Form eines geeigneten Gemisches kontinuierlich in einen gegebenenfalls erhitzten Druckbehälter, der eine intensive Vermischung gewährleistet, so zupumpt, daß
b) die Reaktionstemperatur zwischen 150°C und 350°C liegt und die mittlere Verweilzeit im Druckbehälter 0,1 Sekunden bis 20 Minuten beträgt.

2. Verfahren gemäß Anspruch 1, **dadurch gekennzeichnet, daß** man gleichzeitig den Crotonaldehyd, das Diethylketon und die Lösung oder Suspension der starken Base so in den Druckbehälter pumpt, daß die Reaktionstemperatur 200 bis 300°C beträgt.

3. Verfahren gemäß Anspruch 1, **dadurch gekennzeichnet, daß** man gleichzeitig den Crotonaldehyd, das Diethylketon und die Lösung der starken Base so in den Druckbehälter pumpt, daß die Reaktionstemperatur 220 bis 290°C beträgt.

4. Verfahren gemäß Anspruch 1, **dadurch gekennzeichnet, daß** man gleichzeitig den Crotonaldehyd, das Diethylketon und die Lösung der starken Base so den Druckbehälter pumpt, daß die Reaktionstemperatur 225 bis 280°C beträgt.

5. Verfahren gemäß Anspruch 1, **dadurch gekennzeichnet, daß** die Verweilzeit im Druckbehälter 0,1 Sekunden bis 60 Sekunden beträgt.

6. Verfahren gemäß Anspruch 1, **dadurch gekennzeichnet, daß** man als Lösung einer starken Base konzentrierte wässrige Kalflauge verwendet.

7. Verfahren gemäß Anspruch 1, **dadurch gekennzeichnet, daß** man 0,02 bis 3 Mol einer starken Base verwendet.

## Claims

1. A process for preparing 2,5,6-trimethyl-2-cyclohexen-1-one by reacting diethyl ketone with crotonaldehyde or a compound which is converted under the chosen reaction conditions into crotonaldehyde, in the presence of basic agents at elevated temperature, which comprises
a) simultaneously pumping one mole of crotonaldehyde or one mole of a compound which is converted under the reaction conditions into crotonaldehyde, 5 to 30 moles of diethyl ketone and the aqueous solution or suspension of a strong base, through separate lines or at least partly in the form of a suitable mixture, continuously into a pressure vessel, which is heated where appropriate and which ensures vigorous mixing, in such a way that
b) the reaction temperature is between 150°C and 350°C, and the average residence time in the pressure vessel is 0.1 second to 20 minutes.

2. A process as claimed in claim 1, wherein the crotonaldehyde, the diethyl ketone and the solution or suspension of the strong base are pumped simultaneously into the pressure vessel in such a way that the reaction temperature is 200 to 300°C.

3. A process as claimed in claim 1, wherein the crotonaldehyde, the diethyl ketone and the solution of the strong base are pumped simultaneously into the pressure vessel in such a way that the reaction temperature is 220 to 290°C.

4. A process as claimed in claim 1, wherein the crotonaldehyde, the diethyl ketone and the solution of the strong base are pumped simultaneously into the pressure vessel in such a way that the reaction temperature is 225 to 280°C.

5. A process as claimed in claim 1, wherein the residence time in the pressure vessel is about 0.1 second to 60 seconds.

6. A process as claimed in claim 1, wherein concentrated aqueous potassium hydroxide solution is used as the solution of a strong base.

7. A process as claimed in claim 1, wherein 0.02 to 3 mol of a strong base are used.

## Revendications

1. Procédé pour la préparation de la 2,5,6-triméthyl-cyclohex-2-én-1-one par réaction de la diéthylcétone avec l'aldéhyde crotonique ou un composé qui, dans les conditions de réaction observées, est converti en aldéhyde crotonique, en présence d'agents basiques et à haute température, **caractérisé par le fait que**
a) on envoie par pompe, simultanément, en continu, 1 mol d'aldéhyde crotonique ou 1 mol d'un composé qui, dans les conditions de réaction observées, est converti en aldéhyde crotonique, 5 à 30 mol de diéthylcétone et la solution ou suspension aqueuse d'une base forte, par des conduits d'alimentation séparés ou, en partie au moins, sous forme d'un mélange approprié, dans un récipient sous pression éventuellement chauffé assurant un mélange intensif, dans des conditions telles que
b) la température de réaction se situe entre 150 et 350°C et la durée de passage moyenne dans le récipient sous pression entre 0,1 seconde et 20 minutes.

2. Procédé selon la revendication 1, **caractérisé par le fait que** l'on envoie par pompe, simultanément, l'aldéhyde crotonique, la diéthylcétone et la solution ou suspension de la base forte dans le récipient sous pression dans des conditions telles que la température de réaction se situe entre 200 et 300°C.

3. Procédé selon la revendication 1, **caractérisé par le fait que** l'on envoie par pompe, simultanément, l'aldéhyde crotonique, la diéthylcétone et la solution de la base forte dans le récipient sous pression dans des conditions telles que la température de réaction se situe entre 220 et 290°C.

4. Procédé selon la revendication 1, **caractérisé par le fait que** l'on envoie par pompe, simultanément, l'aldéhyde crotonique, la diéthylcétone et la solution de base forte dans le récipient sous pression, dans des conditions telles que la température de réaction se situe entre 225 et 280°C.

5. Procédé selon la revendication 1, **caractérisé par le fait que** la durée de passage dans le récipient sous pression va de 0,1 s à 60 s.

6. Procédé selon la revendication 1, **caractérisé par le fait que** l'on utilise en tant que solution d'une base forte une lessive de potasse concentrée.

7. Procédé selon la revendication 1, **caractérisé par le fait que** l'on utilise de 0,02 à 3 mol d'une base forte.
